# EUROPEAN PATENT APPLICATION

(11) **EP 4 725 456 A1**
(43) Date of publication of application: **15.04.2026**
(21) Application number: 25172572.7
(22) Date of filing: 25.04.2025
(51) Int. Cl.: A61F 2/30, A61L 27/06, A61C 8/00

(54) **METHOD OF SURFACE TREATMENT OF BONE MATERIALS BY LASER AND NANOSTRUCTURE THEREOF**

(30) Priority: 14.10.2024 TW 113139035
(71) Applicant: Biomate Medical Devices Technology Co., Ltd., Kaohsiung City 821011 (TW); Taiwan Shan Yin International Co., Ltd., Kaohsiung City 806612 (TW)
(72) Inventor: SU, KOU-TSAIR, 821011 Kaohsiung City (TW); SU, YU-JUNG, 821011 Kaohsiung City (TW); CHIANG, TAO, 821011 Kaohsiung City (TW)
(74) Representative: Cabinet Chaillot

(57) **Abstract**

The present disclosure relates to a method of surface treatment of bone materials by laser and nanostructure thereof, wherein the surface of the bone material is titanium metal (3), the laser melts and sputters the titanium metal to form the molten metal titanium. The at least one microchannel (10) of titanium dioxide (TiO₂) is formed during cooling. The at least one microchannel (10) includes at least one micro-hole (12), at least one guiding channel (16), and at least one nano-bump layer (111) formed by the at least one nano-wall (11). The following bone healing and antibacterial effects and purposes will be achieved: A. accelerating the deposition of bone healing related proteins, B. accelerating the attachment of bone cells, C. inducing new blood vessels, D. accelerating the initial calcification and bone mineralization, E. improving the exchange of nutrients, F. stabilizing the surface bone structure, G. forming anti-dental-plaque.

## Description

### FIELD OF THE INVENTION

A bone material, in particular to a method of surface treatment of bone materials by laser and nanostructure thereof.

### BACKGROUND OF THE INVENTION

In the bone material of the prior art, the surface metal of the bone material is processed by a grind group (G) and sandblasting acid etching.

The prior art uses laser to irradiate the surface metal of the bone material to only form an array of pits. The prior art does not disclose forming nano-walls by laser irradiation, nor does it disclose the structure of the pit layout in the guide channel.

In prior art patent No. WO2016171638, the surface preparation method for implants only discloses the steps of using laser irradiation to form pits and applying a small diameter laser to the ridge between the two pits.

### SUMMARY OF THE INVENTION

The present invention discloses a method of surface treatment of bone materials by laser, including: forming molten metallic titanium by continuously ablating a surface of titanium metal of a bone material by irradiating laser at fixed points in a straight axis forward direction along a surface of the titanium metal of the bone material, forming titanium dioxide (TiO₂) during cooling, forming at least one microchannel in the straight axis forward direction by performing molten sputtering on the molten titanium metal, wherein the at least one continuous micro-hole is formed at a bottom side of the at least one microchannel by performing molten sputtering at the surface of the molten titanium metal using the laser, at least one nano-wall of the at least one microchannel is formed at a longitudinal axis side of the at least one microchannel by continuously performing molten sputtering at the at least one micro-hole along the straight axis forward direction using the laser and then performing molten sputtering around the at least one micro-hole to sputter the molten titanium metal onto the surface of the titanium metal; forming at least one guiding channel of the at least one microchannel by performing molten sputtering in between the at least one nano-wall in a direction perpendicular to the irradiated straight axis forward direction of the laser, wherein the guiding channel between the adjacent at least one nano-wall has a first width and a thickness of the nano-wall between the paralleled adjacent at least one guide channel has a pitch; forming a first height of the at least one microchannel by irradiating the laser on the surface of the titanium metal of the bone material along a longitudinal axis direction perpendicular to the irradiated straight axis forward direction, wherein the first height includes a second height of the at least one nano-wall, and a third height of a depth of the micro-hole; forming at least one nano-bump layer with clustered nano-bumps on an upper end surface of the at least one nano-wall by irradiating the laser on the surface of the titanium metal to perform molten sputtering subject to the surface of the titanium metal; and forming at least one wall bottom layer at a lower end surface of the at least one nano-wall by irradiating the surface of the titanium metal to performing molten sputtering subject to the surface of the titanium metal by the laser; wherein, a change in roughness is formed by performing molten sputtering by irradiating the laser on the surface of the titanium metal, a roughness of the at least one nano-bump layer is greater than a roughness formed by the at least one wall bottom layer of the bottom side of the adjacent guiding channel;

wherein, the micro-holes are provided to activate stem cells into osteoblasts, and the osteoblasts form at least one Z-shaped mesh attaching object, which quickly climbs the nano-bump layers between the at least one adjacent nano-wall of the at least one guiding channel.

In one embodiment of the present disclosure, the first width of the guiding channel between adjacent at least one nano-wall is 20 µm to 50 µm, the thickness of the nano-wall between the at least one parallel adjacent guiding channel has the pitch of 10 µm to 30 µm, the first height of at least one microchannel is 20 µm to 60 µm, the second height of the at least one nano-wall is 10 µm to 30 µm, the third height of the depth of the micro-hole is 10 µm to 30 µm, the pitch of the thickness of the at least one nano-wall is 10 µm to 30 µm, a diameter of the at least one micropore is 20 µm to 50 µm, and a spacing of the at least one micro-hole is 30 µm to 50 µm.

In one embodiment of the present disclosure, the laser is switched in a giant pulse generator (Q-Switch) mode, and short pulses are output by a solid-state laser to continuously micro-ablating along the straight axis forward direction through a controlled power intensity.

In one embodiment of the present disclosure, a protein secretion of pre-osteoblasts formed during an osteoblast activation process of the at least one attaching object is selected from at least one of collagen I type α1 chain (COL1A1), decorin (DCN), tumor necrosis factor receptor superfamily member 11B (TNFRSF11B) and secretory phosphoprotein-1 (SPP1).

In one embodiment of the present disclosure, a structure of the at least one microchannel has an ability to resist and inhibit a growth of bacteria inside the at least one microchannel.

In one embodiment of the present disclosure, the titanium metal is titanium or titanium alloy, and the microchannel crystal has a rutile crystal structure.

In one embodiment of the present disclosure, the bone material is a dental implant or a bone screw.

In one embodiment of the present disclosure, the bone material is placed horizontally, and the blood is dripped onto the threaded portion of the bone material, resulting in a contact angle of 0 to 12 degrees.

In one embodiment of the present disclosure, the contact angle of the blood drops to 0 degree in less than 20 seconds.

In one embodiment of the present disclosure, a transmission and exchange channel, for nutrients, blood, serum, and bone calcification, is formed in the guiding channel between the at least one adjacent nano-wall, the micro-hole of the at least one microchannel form a microculture dish, the at least one guiding channel and the at least one micro-hole form a simulated biological environment of the at least one microchannel to promote the formation of new blood vessels.

The present invention also discloses a nanostructure of surface treatment of bone materials by laser, wherein a surface of a bone material is titanium metal, molten metallic titanium is formed by continuously ablating a surface of the titanium metal of the bone material by irradiating laser at fixed points in a straight axis forward direction along a surface of the titanium metal of the bone material, titanium dioxide (TiO₂) is formed during cooling, at least one microchannel in the straight axis forward direction is formed by performing molten sputtering on the molten titanium metal, the at least one microchannel includes: at least one micro-hole arranged on a bottom side of the at least one microchannel along the straight axis forward direction of the laser, wherein the laser continuously melts and sputters the surface of the titanium metal to form the at least one continuous micro-hole of the at least one microchannel; at least one nano-wall arranged on a longitudinal axis side of the at least one microchannel formed along the straight axis forward direction of the laser, wherein after the laser continuously melts and sputters the at least one micro-hole, the laser melts and sputters around the at least one micro-hole onto the surface of the titanium metal to form the at least one nano-wall of the at least one microchannel, on the upper end surface of the at least one nano-wall, at least one nano-bump layer with clustered nano-bumps is formed by melting and sputtering the surface of the titanium metal, and on the lower end surface of the at least one nano-wall, at least one wall bottom layer is formed by melting and sputtering on the surface of the titanium metal; at least one guiding channel arranged between the adjacent at least one nano-wall, wherein, by the laser irradiating along a vertical direction of the irradiated straight axis forward, the laser melts and sputters between the adjacent at least one nano-wall to form the at least one guiding channel of the at least one microchannel; wherein, the guiding channel between the adjacent at least one nano-wall has a first width and a thickness of the nano-wall between the parallel adjacent at least one guiding channel has a pitch; wherein a first height of the at least one microchannel includes a second height of the at least one nano-wall and a third height of a depth of the micro-hole; wherein, a change in roughness is formed by performing molten sputtering through irradiating the laser on the surface of the titanium metal, a roughness of the at least one nano-bump layer is greater than a roughness formed by the at least one wall bottom layer of the bottom side of the adjacent guiding channel; wherein the micro-holes are provided to activate stem cells into osteoblasts, and the osteoblasts form at least one Z-shaped mesh attaching object, which quickly climbs the nano-bump layers of the at least one nano-wall between the adjacent at least one guiding channel.

In one embodiment of the present disclosure, the first width of the guiding channel between adjacent at least one nano-wall is 20 µm to 50 µm, the thickness of the nano-wall between the at least one parallel adjacent guiding channel has the pitch of 10 µm to 30 µm, the first height of at least one microchannel is 20 µm to 60 µm, the second height of the at least one nano-wall is 10 µm to 30 µm, the third height of the depth of the micro-hole is 10 µm to 30 µm, the pitch of the thickness of the at least one nano-wall is 10 µm to 30 µm, a diameter of the at least one micropore is 20 µm to 50 µm, and a spacing of the at least one micro-hole is 30 µm to 50 µm.

In one embodiment of the present disclosure. the laser is switched in a giant pulse generator (Q-Switch) mode, and short pulses are output by a solid-state laser to continuously micro-ablating along the straight axis forward direction through a controlled power intensity.

In one embodiment of the present disclosure, a protein secretion of pre-osteoblasts formed during an osteoblast activation process of the at least one attaching object is selected from at least one of collagen I type α1 chain (COL1A1), decorin (DCN), tumor necrosis factor receptor superfamily member 11B (TNFRSF11B) and secretory phosphoprotein-1 (SPP1).

In one embodiment of the present disclosure, a structure of the at least one microchannel has an ability to resist and inhibit a growth of bacteria inside the at least one microchannel.

In one embodiment of the present disclosure, the titanium metal is titanium or titanium alloy, and the microchannel crystal has a rutile crystal structure.

In one embodiment of the present disclosure, the bone material is a dental implant or a bone screw.

In one embodiment of the present disclosure, the bone material is placed horizontally, and the blood is dripped onto the threaded portion of the bone material, resulting in a contact angle of 0 to 12 degrees.

In one embodiment of the present disclosure, the contact angle of the blood drops to 0 degree in less than 20 seconds.

In one embodiment of the present disclosure, a transmission and exchange channel, for nutrients, blood, serum, and bone calcification, is formed in the guiding channel between the at least one adjacent nano-wall, the micro-hole of the at least one microchannel forms a microculture dish, the at least one guiding channel and the at least one micro-hole form a simulated biological environment of the at least one microchannel to promote the formation of new blood vessels.

The present disclosure provides a method of surface treatment of bone materials by laser and nanostructure thereof. The present disclosure has the nano-bump layer, the wall bottom layer, micropores of the nano-wall, and the guiding channel located on the titanium surface, which enhance protein adhesion and cell attachment. The stretch of the pre-osteoblast provides more climbing points and provides the ability of forming the antibiofilm, thereby increasing bone integration, osteoblast proliferation and adhesion, proliferation and osteogenic differentiation. In addition, the surface treatment of the deeper microchannels on the titanium surface by laser of the present disclosure may create more space, promote sufficient nutrition supplementation of pre-osteoblasts, and may help the pre-osteoblast maturation process, including proliferation, osteogenic differentiation and extracellular mineralization. The present disclosure specifically achieves the purpose of accelerating bone healing speed and the bone integration effect of stable adhesion between bone and human tissue.

The present disclosure provides a method of surface treatment of bone materials by laser and nanostructure thereof, thereby establishing a work module of laser-processed microchannels with attached micro-holes and nanostructures. The nanostructure may increase the following bone healing and antibacterial indicators: A. accelerating the deposition of bone healing-related proteins, B. accelerating the attachment of bone cells, C. inducing new blood vessels, D. accelerating initial calcification and bone mineralization, E. improving nutrient exchange, F. stabilizing the surface bone structure, G. forming the anti-plaque.

### BRIEF DESCRIPTION OF THE DRAWINGS

FIG. 1 is a schematic diagram illustrating the nanostructure of the bone material with the surface treated by laser in the present disclosure.
FIG. 2 is a schematic diagram illustrating the implant of the bone material with the surface treated by laser corresponding to periodontal ligament in the present disclosure.
FIG. 3 is a schematic diagram illustrating the implant of the bone material with the surface treated by laser applied to the angle corresponding to the laser irradiation and implant shape in the present disclosure.
FIG. 4 is a schematic diagram illustrating the bone screw of the bone material with the surface treated by laser applied to the palmar scaphoid bone in the present disclosure.
FIG. 5 is a schematic diagram illustrating the implant of the bone material with the surface treated by laser applied to blood contact angle and adsorption angle in the present disclosure.
FIG. 6 is a schematic diagram illustrating the nanostructure of the bone material with the surface treated by laser compared with sandblasting acid etching in the present disclosure.
FIG. 7 is a schematic diagram illustrating the scanning electron microscope images of the nanostructure of the bone material with the surface treated by laser compared with grind plate and sandblasted acid etching titanium plate in the present disclosure.
FIG. 8 is a schematic diagram illustrating the nanostructure of the bone material with the surface treated by laser compared with sandblasted acid etching in the present disclosure.
FIG. 9 is a schematic diagram illustrating the serum adhesion of the bone material with the surface treated by laser compared with sandblasted acid etching in the present disclosure.
FIG. 10 is a schematic diagram illustrating the cell number of the bone material with the surface treated by laser compared with grind and sandblasted acid etching in the present disclosure.
FIG. 11-1 is a schematic diagram illustrating the ratio of mRNA expression of the bone material with the surface treated by laser compared with grind and sandblasted acid etching in the present disclosure.
FIG. 11-2 is a schematic diagram illustrating the purified cell protein lysates used for Western blot assays with antibodies against (A) COL1A1, (B) DCN, (C) TNFRSF11B, (D) SPP1 and β-actin in the bone material with the surface treated by laser of the present disclosure.
FIG. 12 is a schematic diagram illustrating the concentrations of COL1A1, DCN, and TNFRSF11B of the bone material with the surface treated by laser compared with grind and sandblasted acid etching in the present disclosure.
FIG. 13 is a schematic diagram illustrating the mineralization of the bone material with the surface treated by laser compared with grind and sandblasted acid etching in the present disclosure.
FIG. 14 is a schematic diagram illustrating the guiding channel of the present disclosure has enough first width of 20 µm to 50 µm, and the surface of the microchannel of the present disclosure has better bone cell growth, compared with the implant having only 10 µm in the prior art.
FIG. 15 is a schematic diagram illustrating the tightly integrated interface structure of implant and bone, which is completely different from the separated interface structure of the SLA implant and bone.
FIG. 16 is a schematic diagram illustrating the formation, amount of biofilm, and the amount of dental plaque components of a total of five surfaces, namely, the nanostructured surface of the microchannel of the present disclosure, the laser surface with dot-matrix pits, the machined surface, the polished surface, and the sandblasted surface.

### DETAILED DESCRIPTION OF THE INVENTION

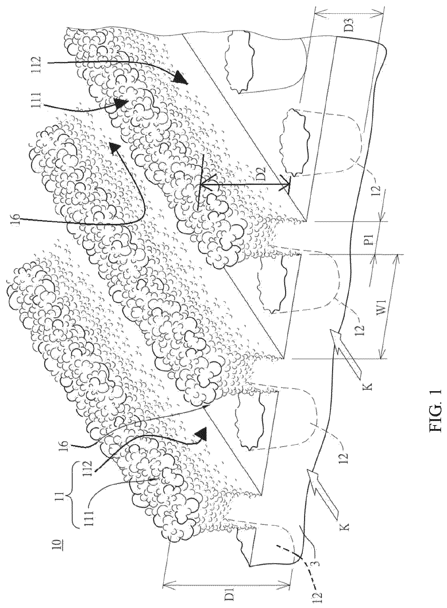

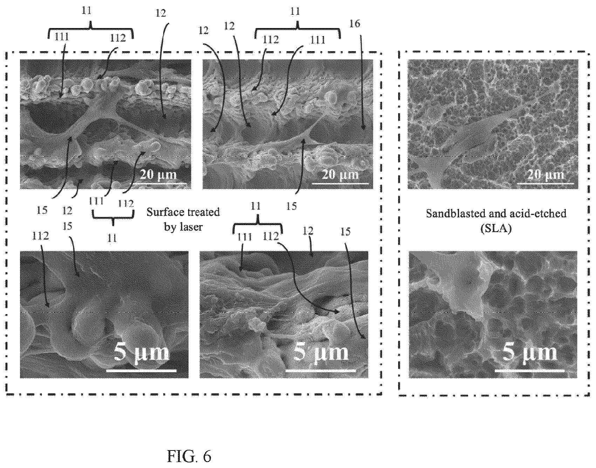

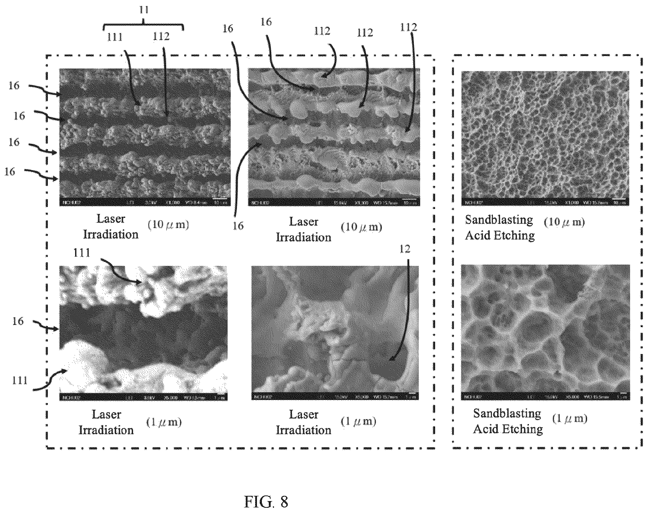

As shown in FIG. 1, FIG. 6, FIG. 7, and FIG. 8, an embodiment of the present disclosure is a method of surface treatment of bone materials by laser. The at least one microchannel 10 includes at least one micro-hole 12, at least one nano-wall 11 and at least one guiding channel 16. The at least one nano-wall 11 includes at least one nano-bump layer 111 and at least one wall bottom layer 112. The steps include forming molten metallic titanium by continuously ablating a surface of the titanium metal 3 of the bone material by irradiating laser at fixed points in a straight axis forward direction along the surface of the titanium metal 3 of the bone material, and forming at least one microchannel 10 in the straight axis forward direction by performing molten sputtering on the molten titanium metal 3, wherein at least one continuous micro-hole 12 is formed at a bottom side of the at least one microchannel 10 by performing molten sputtering at the surface of the molten titanium metal 3 using the laser, wherein at least one nano-wall of the at least one microchannel 10 is formed at a longitudinal axis side of the at least one microchannel 10 by continuously performing molten sputtering at the at least one micro-hole 12 along the straight axis forward direction using the laser and then performing molten sputtering around the at least one micro-hole 12 to sputter molten metal onto the surface of the titanium metal 3; forming at least one guiding channel 16 of the at least one microchannel 10 by performing molten sputtering in between the at least one nano-wall 11 in a direction perpendicular to the irradiated straight axis forward direction of the laser, wherein the guiding channel 16 between the adjacent at least one nano-wall 11 has a first width W1 and a thickness of the nano-wall 11 between the paralleled adjacent at least one guide channel 16 has a pitch P1; forming a first height D1 of the at least one microchannel 10 by irradiating the laser on the surface of the titanium metal 3 of the bone material along a longitudinal axis direction perpendicular to the irradiated straight axis forward direction, wherein the first height D1 includes a second height D2 of the at least one nano-wall 11, and a third height D3 of a depth of the micro-hole 12; forming at least one nano-bump layer 111 with clustered nano-bumps on an upper end surface of the at least one nano-wall 11 by irradiating the laser on the surface of the titanium metal 3 to perform molten sputtering subject to the surface of the titanium metal 3; and forming at least one wall bottom layer 112 at a lower end surface of the at least one nano-wall 11 by irradiating the surface of the titanium metal 3 to performing molten sputtering subject to the surface of the titanium metal 3 by the laser; wherein, a change in roughness is formed by performing molten sputtering by irradiating the laser on the surface of the titanium metal 3, a roughness of the at least one nano-bump layer 111 is greater than a roughness formed by the at least one wall bottom layer 112 of the bottom side of the adjacent guiding channel 16; wherein, the micro-holes 12 are provided to activate cells into osteoblasts, and the osteoblasts form at least one Z-shaped mesh attaching object 15, which quickly climbs the nano-bump layers 111 between the at least one adjacent nano-wall 11 of the at least one guiding channel 16. The laser irradiation scan direction K. A cell attaching area is formed at a junction of the at least one nano-bump layer 111 on the upper end surface of the at least one nano-wall 11 and the at least one wall bottom layer 112 on the lower end surface of the at least one nano-wall 11, thereby activating the cells into osteoblasts. The laser is continuously irradiated at fixed points on the surface of the titanium metal 3 of the bone material to continuously ablate and form the molten metallic titanium. Titanium dioxide is formed when the titanium is cooled after the irradiation. When the irradiation process has not cooled down, there will be no titanium dioxide, but molten metallic titanium.

As shown in FIG. 1, FIG. 6, FIG. 7 and FIG. 8, an embodiment of the present disclosure relates to a method of surface treatment of bone materials by laser and nanostructure thereof, wherein a titanium dioxide oxide layer is formed on the surface of the titanium metal 3 to optimize the adhesion and regeneration of blood and bone cells. The nanostructure of the present disclosure is formed by the laser. When irradiated by laser emission, the irradiated area on the surface of the titanium metal 3 continuously melt, sputter and ablate, and combine with oxygen atoms in the air to form an oxide layer of a titanium dioxide, and the thickness of which is determined by the adjustment of the laser parameters. In the present disclosure, the bottom of the microchannel 10 is measured by X-ray photoelectron spectroscopy, the thickness of the oxide layer at the bottom of the micro-hole 12 is about 45 nm, and the thickness of the oxide layer at the upper end of the microchannel 10 (until the upper end of the nano-wall 11) is about 110 nm. Compared with the surface structures in the other prior arts, the oxide layer of the surface structure in the present disclosure is much higher.

As shown in FIG. 1, FIG. 6, FIG. 7 and FIG. 8, an embodiment of the present disclosure relates to a method of surface treatment of bone materials by laser and nanostructure thereof, wherein the first width W1 of the guiding channel 16 between adjacent at least one nano-wall 11 is 20 µm to 50 µm, the thickness of the nano-wall 11 between the at least one parallel adjacent guiding channel 16 has the pitch P1 of 10 µm to 30 µm, the first height D1 of at least one microchannel 10 is 20 µm to 60 µm, the second height D2 of the at least one nano-wall 11 is 10 µm to 30 µm, the third height D3 of the depth of the micro-hole 12 is 10 µm to 30 µm, the pitch P1 of the thickness of the at least one nano-wall 11 is 10 µm to 30 µm, a diameter of the at least one micropore 12 is 20 µm to 50 µm, and a spacing of the at least one micro-hole 12 is 30 µm to 50 µm.

As shown in FIG. 1, FIG. 6, FIG. 7 and FIG. 8, an embodiment of the present disclosure relates to a method of surface treatment of bone materials by laser and nanostructure thereof, wherein the laser is switched in giant pulse generator (Q-Switch) mode, and short pulses are output by a solid-state laser to continuously micro-ablating along the straight axis forward direction through controlled power intensity.

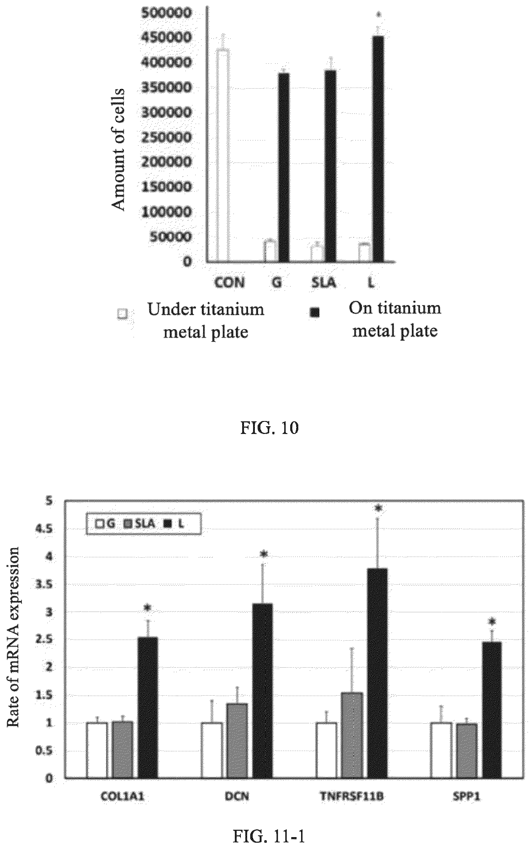

As shown in FIG. 11-1 to FIG. 12, an embodiment of the present disclosure relates to a method of surface treatment of bone materials by laser and nanostructure thereof, wherein a protein secretion of pre-osteoblasts formed during an osteoblast activation process of the at least one attaching object 15 is selected from at least one of collagen I type α1 chain (COL1A1), decorin (DCN), tumor necrosis factor receptor superfamily member 11B (TNFRSF11B) and secretory phosphoprotein-1 (SPP1).

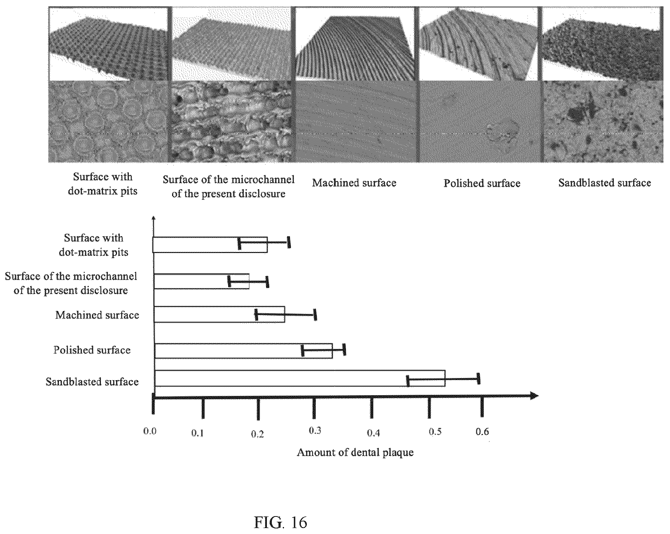

As shown in FIG. 16, an embodiment of the present disclosure relates to a method of surface treatment of bone materials by laser and nanostructure thereof, wherein a structure of the at least one microchannel 10 has an ability to resist and inhibit a growth of bacteria inside the at least one microchannel 10.

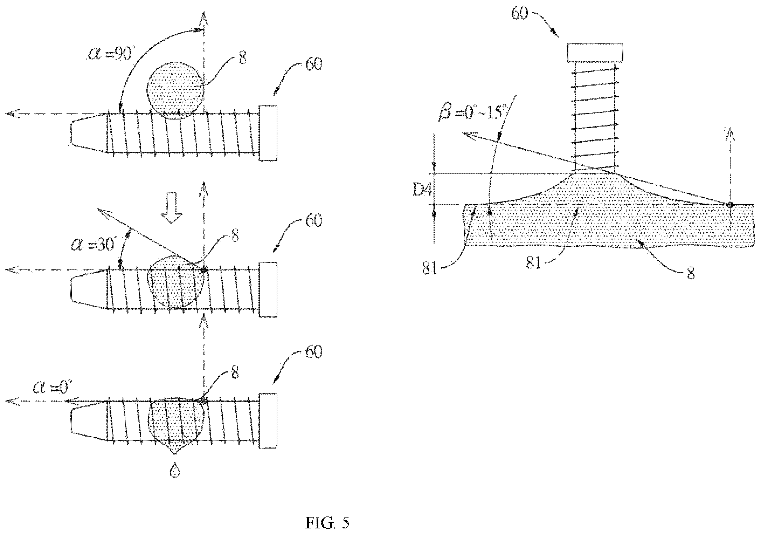

As shown in FIG. 5, an embodiment of the present disclosure relates to a method of surface treatment of bone materials by laser and nanostructure thereof, wherein the titanium metal 3 is titanium or titanium alloy, and the microchannel 10 crystal has a rutile crystal structure. The bone material is a dental implant or a bone screw. The bone material is placed horizontally, and the blood is dripped onto the threaded portion of the bone material, resulting in a contact angle of 0 to 12 degrees. It takes less than 20 seconds for the contact angle of the blood to drop to 0 degree.

As shown in FIG. 1 to FIG. 9, an embodiment of the present disclosure relates to a method of surface treatment of bone materials by laser and nanostructure thereof, wherein a transmission and exchange channel, for nutrients, blood, serum, and bone calcification, is formed in the guiding channel 16 between the at least one adjacent nano-wall 11, the micro-hole 12 of the at least one microchannel 10 forms a microculture dish, the at least one guiding channel 16 and the at least one micro-hole 12 form a simulated biological environment of the at least one microchannel 10 to promote the formation of new blood vessels.

As shown in FIG. 1 to FIG. 9, an embodiment of the present disclosure relates to a method of surface treatment of bone materials by laser and nanostructure thereof. The present disclosure may improve the exchange of nutrients. The microchannel 10 provides an excellent surface area for monolayer cell attachment, while achieving good temperature regulation and rapid exchange of liquid media. The present disclosure is suitable for the growth and differentiation of the osteoblasts and endothelial cells inside and outside the microchannel 10 with nanostructure.

As shown in FIG. 1 to FIG. 9, an embodiment of the present disclosure relates to a nanostructure of surface treatment of bone materials by laser, wherein a surface of the bone material is titanium metal 3. The laser continuously irradiates a surface of the titanium metal 3 of the bone material to continuously ablate the formed molten titanium metal. The titanium metal 3 is melted and sputtered to form at least one microchannel 10 in the straight axis forward direction. The at least one microchannel 10 includes at least one micro-hole 12, which is arranged on a bottom side of the at least one microchannel 10 along the straight axis forward direction of the laser, and the laser continuously melts and sputters the surface of the titanium metal 3 to form the at least one continuous micro-hole 12 of the at least one microchannel 10. The at least one microchannel 10 also includes at least one nano-wall 11 arranged on a longitudinal axis side of the at least one microchannel 10 and is formed along the straight axis forward direction of the laser. After the laser continuously melts and sputters the at least one micro-hole 12, the laser melts and sputters around the at least one micro-hole 12 onto the surface of the titanium metal 3 to form the at least one nano-wall 11 of the at least one microchannel 10. On the upper end surface of the at least one nano-wall 11, at least one nano-bump layer 111 with clustered nano-bumps is formed by melting and sputtering the surface of the titanium metal 3, and on the lower end surface of the at least one nano-wall 11, at least one wall bottom layer 112 is formed by melting and sputtering on the surface of the titanium metal 3. The at least one microchannel 10 also includes at least one guiding channel 16 arranged between the adjacent at least one nano-wall 11. By irradiating the laser along a vertical direction of the irradiated straight axis forward, the laser melts and sputters between the adjacent at least one nano-wall 11 to form the at least one guiding channel 16 of the at least one microchannel 10. The guiding channel 16 between the adjacent at least one nano-wall 11 has a first width W1 and a thickness of the nano-wall 11 between the parallel adjacent at least one guiding channel 16 has a pitch P1. A first height D1 of the at least one microchannel 10 includes a second height D2 of the at least one nano-wall 11, and a third height D3 of a depth of the micro-hole 12. Wherein, a change in roughness is formed by performing molten sputtering through irradiating the laser on the surface of the titanium metal 3, a roughness of the at least one nano-bump layer 111 is greater than a roughness formed by the at least one wall bottom layer 112 of the bottom side of the adjacent guiding channel 16. Wherein, the micro-holes 12 are provided to activate cells into osteoblasts, and the osteoblasts form at least one Z-shaped mesh attaching object 15, which quickly climbs the nano-bump layers 111 of the at least one nano-wall 11 between the adjacent at least one guiding channel 16.

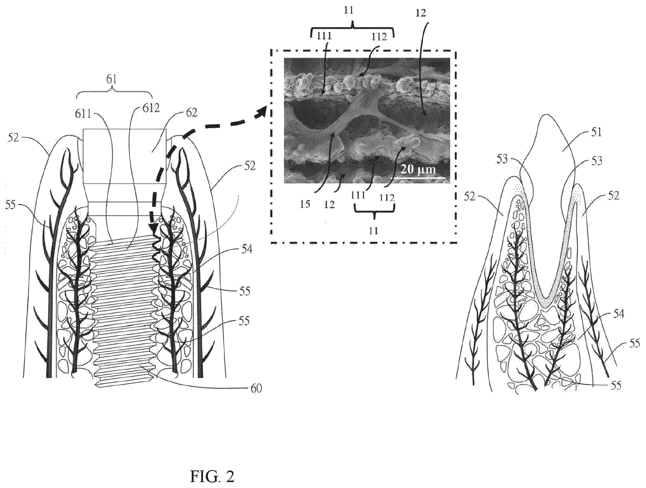

As shown in FIG. 2, an embodiment of the present disclosure relates to a method of surface treatment of bone materials by laser and nanostructure thereof, wherein a periodontal ligament 53 is in between the tooth 51 and the alveolar bone 54, the gum 52 has blood vessels 55, and the periodontal ligament 53 provides nutrients and blood to the tooth 51 and the alveolar bone 54 via the blood vessels 55. The dental implant 60 of surface treatment of bone materials by laser in the present disclosure has a microchannel 10, which can simulate the function of the periodontal ligament 53. The microchannel 10 structure can increase bone healing and antibacterial indicators, accelerate the deposition of bone healing related proteins, accelerate the attachment of bone cells, induce new blood vessels, accelerate initial calcification and bone mineralization, improve the exchange of nutrients, stabilize the surface bone structure, and resist the formation of dental plaque. The microchannel 10 on the laser surface area in the present disclosure provides nutrients and blood to the alveolar bone through blood vessels, thereby accelerating bone healing with the alveolar bone.

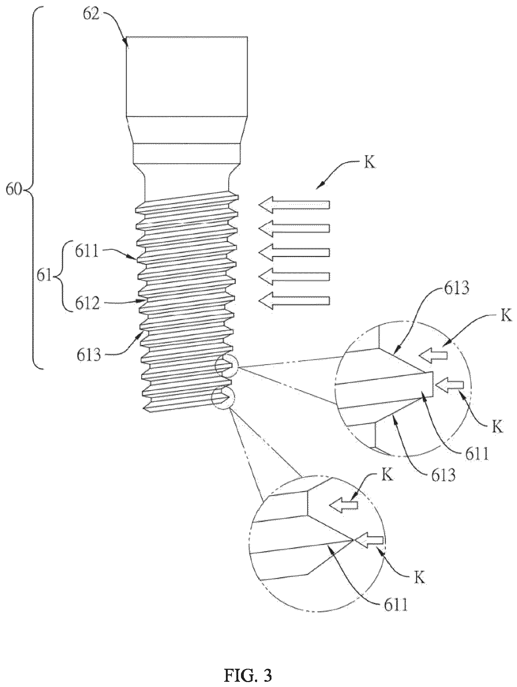

As shown in FIG. 3, an embodiment of the present disclosure relates to a method of surface treatment of bone materials by laser and nanostructure thereof, wherein an implant 60 has a joint end 62 and a threaded portion 61. The threaded portion 61 includes a threaded protrusion portion 611 and a threaded recess portion 612, wherein if a surface layer 613 of the threaded portion is parallel to a laser irradiation scanning direction K, the laser irradiation cannot scan, melt and sputter the surface layer 613. Therefore, the surface layer 613 of the threaded protrusion portion 611 and the threaded recess portion 612 can be trapezoidal, such that the surface layer 613 forms a non-parallel angle with the laser irradiation scanning direction K, and the laser irradiation may effectively melt and sputter the surface layer 613 to form specific effective structures of the microchannel 10, including the nano-bump layer 111 of the nano-wall 11, the wall bottom layer 112 of the nano-wall 11, the micro-holes 12 and the guiding channels 16 located on the surface of the titanium metal 3.

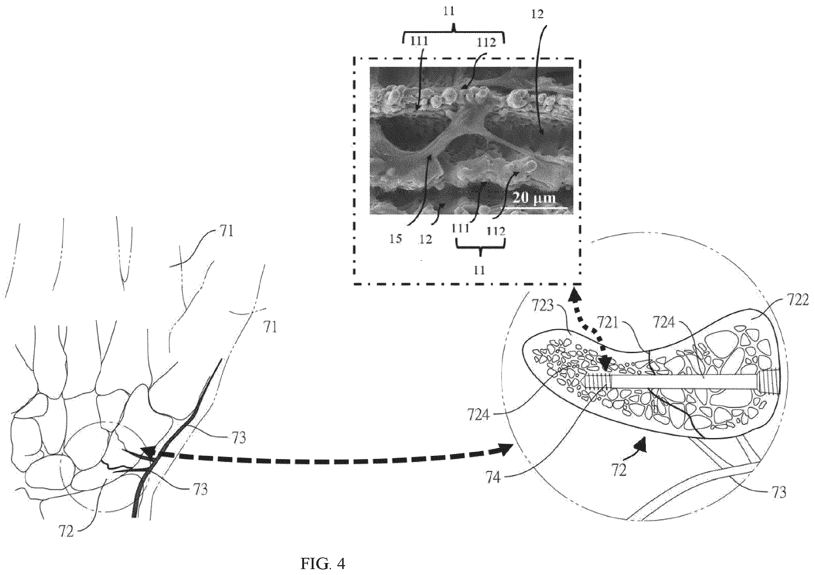

As shown in FIG. 4, an embodiment of the present disclosure relates to a method of surface treatment of bone materials by laser and nanostructure thereof, wherein after a finger 71 and a scaphoid bone 72 in the palm are fractured due to sports injuries, a fracture crack 721 of the scaphoid bone 72 breaks the scaphoid bone 72 into a front portion 722 and a rear portion 723. The front portion 722 has blood vessels 73 to supply bone marrow 724, while the rear portion 723 has no blood vessels 73 to supply bone marrow 724, which is, therefore, prone to necrosis, and the fracture cannot be healed easily. The bone nail 74 in the present disclosure forms the specific effective structures of the microchannel 10 on the titanium surface of the bone nail 74, including the nano-bump layer 111 of the nano-wall 11, the wall bottom layer 112, the micro-holes 12 and the guiding channels 16. This structure can increase bone healing and antibacterial indicators, accelerate the deposition of bone healing related proteins, accelerate the attachment of bone cells, induce new blood vessels, accelerate initial calcification and bone mineralization, improve the exchange of nutrients, stabilize the surface bone structure, and resist the formation of dental plaque. Therefore, the rear portion 723 has a microchannel 10 to supply blood and nutrients to the bone marrow 724, such that the scaphoid 72 may not necrotize easily, and the fracture may be healed faster.

As shown in FIG. 5, an embodiment of the present disclosure relates to a method of surface treatment of bone materials by laser and nanostructure thereof, a blood drop test implant 60 of blood 8 is placed vertically, and an adsorption angle β of 0 to 15 degrees is generated by the blood 8 and the horizontal line 81 with a fourth height D4. The implant 60 is placed vertically and the contact angle α drops to 0 degree in less than only 20 seconds. Because the implant 60 in the present disclosure has the specific effective structures of the microchannel 10 located on the titanium surface of the implant 60, including the nano-bump layer 111 of the nano-wall 11, the wall bottom layer 112 of the nano-wall 11, the micro-holes 12, and the guiding channels 16, the adsorption angle β of the present disclosure is greater than that of the general implants treated by grind group (G) and sandblasting acid etching group (SLA). The contact angle α of the present disclosure is smaller than that of the general implants in the grind group (G) and sandblasting acid etching group (SLA). In addition, when the microspheres flow over the surface of the nanostructure of the present disclosure, the microspheres can flow at a significantly higher speed, and sufficiently to cause a significant difference in cell adhesion and protein absorption. In the laser-treated structure with hydrophilic micro/nano-holes, the cell adhesion, proliferation, differentiation and prostaglandin E2 production on the surface structure of the present disclosure are higher than the cell adhesion, proliferation, differentiation and prostaglandin E2 production on the polished titanium metal 3 and the titanium metal 3 surface treated by SLA. Laser treatment can produce the hydrophilic micro/nano-hole porous titanium surface of the present disclosure. The structural surface characteristics of the present disclosure may enhance the adhesion, proliferation and differentiation of MG-63 cells. In addition, the scanning electron microscopy (SEM) showed that the dental implant with structural surface characteristics of the present disclosure has strong wear resistance and only a small amount of damage may be occurred during repeated insertion and removal. These damages include spalling, pitting and deformation. In comparison, the SLA implant surface has more pits, deformation, and fusion areas than the present disclosure. The defects in the prior art are particularly likely to be found at the tip thread portion corresponding to the self-tapping design. The surface of the present disclosure has no obvious sharp bumps and depressions under a microscopic level, and this is why the surface of the present disclosure changes less and is more durable. Therefore, under high torque, the macro-nano roughness of the present disclosure may not be damaged easily. However, the surface of the SLA may usually have sharp bumps and depressions. Therefore, when subjected to compression force, these bumps and depressions may inevitably deform and may be damaged. The surface of the present disclosure is more resistant to pressure in physical structure. In clinical application, the implant of the present disclosure can be removed and reinserted without endangering the implant.

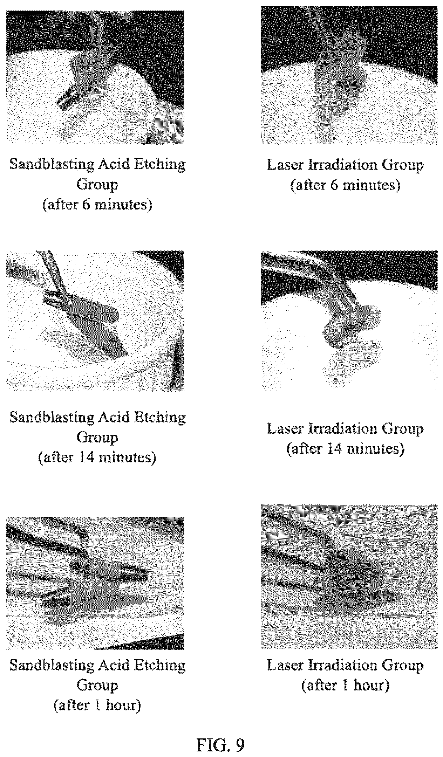

As shown in FIG. 9 to FIG. 12, an embodiment of the present disclosure relates to a method of surface treatment of bone materials by laser and nanostructure thereof. The present disclosure may accelerate the deposition of proteins related to bone healing. The present disclosure uses high laser energy to produce a high oxide layer (45-110 nm) on the surface of the titanium metal 3 structure of the present disclosure. The titanium dioxide oxide layer can optimize blood and serum compatibility, thereby providing an environment for attaching the fibrin to facilitate the formation of a temporary stroma, which is very important in the early stages of bone healing. As shown in FIG. 9 , the serum viscosity test of the present disclosure showed that the serum viscosity of the implant 60 in the present disclosure is greater than that of the general implants treated by the grind group (G) and sandblasting acid etching group (SLA) because the implant 60 in the present disclosure has the specific effective structures of the microchannel 10 on the titanium surface of the implant 60, including the nano-bump layer 111 of the nano-wall 11, the wall bottom layer 112, the micro-holes 12 and the guiding channels 16 located on the titanium surface of the implant 60.

As shown in FIG.1, an embodiment of the present disclosure relates to a method of surface treatment of bone materials by laser and nanostructure thereof, wherein at the laser surface area, the melting and sputtering depth of the first height D1, the second height D2 and the third height D3 of the microchannel 10 cannot be too shallow. Because it would be difficult to provide nutrients, blood and serum if the guiding channel 16 doesn't have enough depth. The first width W1 cannot be formed too wide, otherwise the attaching object 15 may not climb easily. For example, the pre-bone cell protein secretion cannot easily climb and form between the at least one nano-wall 11 of the guiding channel 16 and cannot easily form the Z-shaped mesh climb. The first width W1 of the microchannel 10 cannot be formed too narrow otherwise the amount of the attaching object 15 would be less. It is not allowed for the microchannel 10 to not to be sputtered and not to have the nano-wall 11 with a special nano-bump layer 111, otherwise in the upper layer of the guiding channel 16, the mesh cells of the attaching object 15 or pre-osteoblasts may not easily adhere to the nano-bump layer 111 and the pre-osteoblast protein secretions may not easily be secreted. The microchannel 10 has a plurality of molten sputtered deep micro-holes 12 with the third height D3, which may simulate biological environment proliferation and the micro culture dish effect. The microchannel of present disclosure is scanned at the same position vertically and scanned along the horizontal direction to produce the optimized structure mentioned above by laser frequency, energy intensity, pulse (frequency), specific unit area/unit time/unit pulse/unit energy.

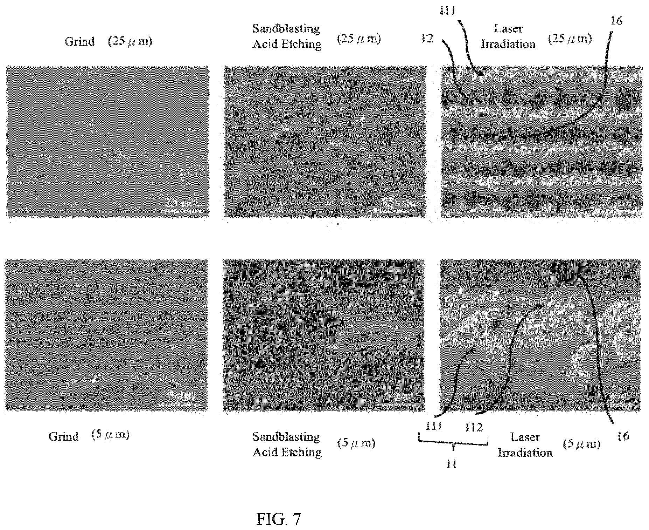

As shown in FIG.7 and FIG. 10, an embodiment of the present disclosure relates to a method of surface treatment of bone materials by laser and nanostructure thereof. Scanning electron microscope images of the titanium plates, which have been treated by the grind group (G), sandblasting acid etching group (SLA), and laser irradiation group (L), are provided, respectively. As shown in FIG. 10, pre-osteoblasts and palatal mesenchyma cells of human embryos have been proven to differentiate into osteoblasts on titanium plates. In the present disclosure, 2.3 × 10⁵ pre-osteoblasts are cultured for three days on titanium plates, which have been treated by grind group (G), sandblasting acid etching group (SLA), and laser irradiation group (L), respectively. Effect of three groups of the modified titanium surfaces on the growth of pre-osteoblasts is according to the proliferation rate of the pre-osteoblasts. The number of cells in the laser irradiation group (L) of the present disclosure is higher than that of the grind group (G) and the sandblasting acid etching group (SLA). The present disclosure may accelerate bone cell attachment, and the structure of the microchannel 10 of the present disclosure can maximize the surface area for monolayer cell attachment while achieving good temperature regulation and rapid exchange of liquid culture medium. The microchannel 10 of the present disclosure acts as a bioreactor, generating a local flow environment that makes many cell types, such as osteoblasts (bone cells) and endothelial cells (the lining of blood vessels), evolve to prefer the continuous exposure of the shear stress of 0.1 dyne/cm, and they only show normal phenotypes in the environment of the microchannel 10 provided by the present disclosure.

As shown in FIG. 11-1, an embodiment of the present disclosure relates to a method of surface treatment of bone materials by laser and nanostructure thereof. The present disclosure may accelerate bone cell attachment. Compared to the surfaces treated by the SLA group and the G group, the surface of the titanium metal 3 of the laser-treated microchannel 10 structure of the present disclosure increases bone integration ability. The laser-treated titanium surface with the channel structure in the present disclosure enhances the integration of osteoblast and bone before maturation. According to the qPCR results of the present disclosure, regarding the pre-osteoblasts on the surface which is treated by the laser irradiation group (L), the mRNA, protein expression/secretion ratio and extracellular mineralization of COL1A1, DCN, TNFRSF11B and SPP1 are significantly increased. Wherein, the minimum information for publication of quantitative real-time PCR experiments (MIQE) of experiment guide for publishing the quantitative real-time polymerase chain reaction (PCR) is abbreviated to qPCR for quantitative real-time PCR. The titanium surface treated by the present disclosure upregulated the expression of osteogenic differentiation markers in the pre0osteoblast obtained by qPCR assay. The 3 × 10⁵ pre-osteoblasts are respectively cultured on the titanium plates with the surfaces treated by the grind group (G), sandblasting acid etching group (SLA), and laser irradiation group (L) for 72 hours. Purified cellular mRNA is used for primer sets for COL1A1, DCN, TNFRSF11B and SPP1 of the qPCR assays. Osteogenic differentiation markers are, for example, COL1A1, DCN, TNFRSF11B and SPP1. Wherein, collagen type I alpha 1 (COL1A1) is the main component of collagen type I and is widely distributed in the interstitium of parenchymal organs and connective tissues throughout the body. Collagen plays an important role in maintaining tissue development and homeostasis. Decorin (DCN) is a protein encoded by the DCN gene in humans. Decorin (DCN) is a kind of proteoglycan. Decorin (DCN) is a kind of small cell or pericellular matrix proteoglycan which is structurally closely related to biglycan protein. Decorin (DCN) is the result of gene duplication. This protein is a component of connective tissue which binds to the type I collagen fibrils and plays a role in matrix assembly. Decorin (DCN) is named because it "decorates" the collagen type I and it interacts with the "d" and "e" bands of the fibrils of collagen. Decorin affects fiber formation and is a myokine. In this effect, decorin binds to myostatin to promote muscle hypertrophy. Wherein, tumor necrosis factor receptor superfamily member 11B (TNFRSF11B) is called osteoprotegelin (OPG), also known as osteoclastogenesis inhibitory factor (OCIF). Osteoprotegerin (OPG) was originally discovered to be a novel secreted tumor necrosis factor receptor (TNFR) related protein that plays a role in bone density regulation. Wherein, secreted phosphoprotein-1 (SPP1), also known as osteopontin (OPN), is a glycoprotein associated bone formation. Secreted phosphoprotein-1 (SPP1) plays a role in anchoring osteoclasts to the bone remodeling matrix by binding to the vitronectin (VTN) receptor. Wherein, vitronectin (VTN) is a multifunctional glycoprotein with multiple physiological functions, which exists in plasma and extracellular matrix. Vitronectin (VTN) mainly binds to integrin receptors through the RGD sequence and participates in cell attachment, spreading and migration. Vitronectin (VTN) is also widely used in the maintenance and expansion of pluripotent stem cells. Vitronectin (VTN) has more functions in the nervous system because it is involved in neural differentiation, neurotrophy and neurogenesis, as well as regulating axon size, supporting and guiding neurite extension. In addition, vitronectin (VTN) has been proven to play a key role in protecting the brain as it can reduce the permeability of the blood-brain barrier by interacting with integrin receptors in vascular endothelial cells.

As shown in FIG. 11-1, an embodiment of the present disclosure relates to a method of surface treatment of bone materials by laser and nanostructure thereof. The present disclosure may accelerate bone cell attachment and cause pre-osteoblasts to express/secrete more COL1A1, DCN, TNFRSF11B and SPP1 proteins. In addition, the titanium surface treated by the laser irradiation group (L) may upregulate the expression of osteogenic differentiation markers in pre-osteoblasts as obtained by Western blot assay.

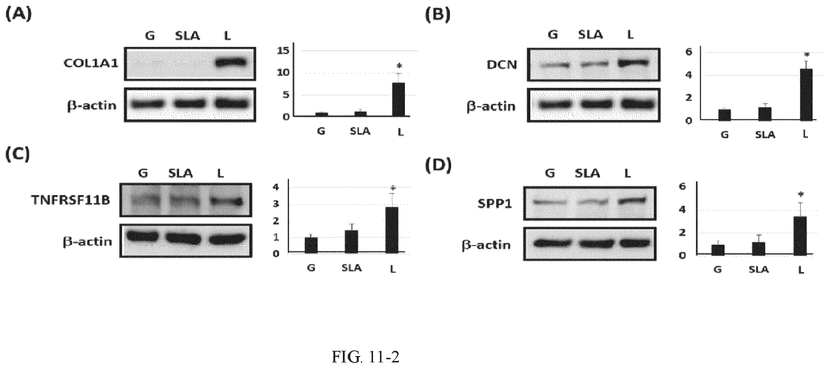

As shown in FIG. 11-2, an embodiment of the present disclosure relates to a method of surface treatment of bone materials by laser and nanostructure thereof. The 2.3 × 10⁵ pre-osteoblasts are incubated on the titanium plates with surfaces treated by G, SLA and L for 72 hours. The purified cell protein lysates are used for Western blot assays of (A) COL1A1, (B) DCN, (C) TNFRSF11B, (D) SPP1, and β-actin antibodies. Regarding the pre-osteoblasts on the surface treated by laser irradiation group (L), the expression of COL1A1, DCN, TNFRSF11B and SPP1 by Western blot assay are significantly increased.

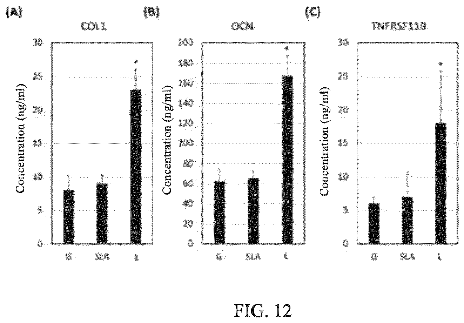

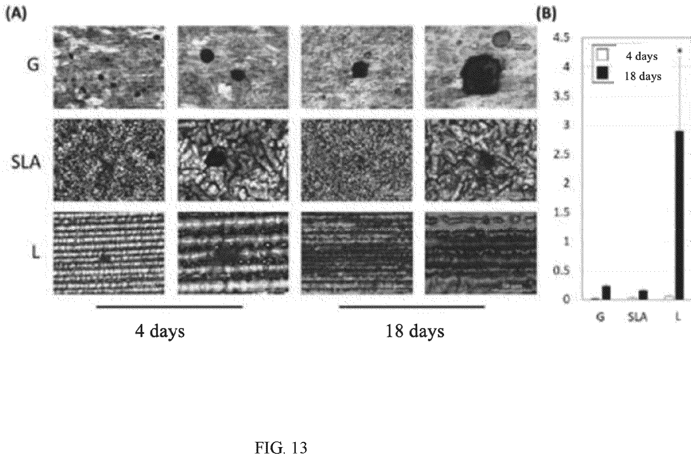

As shown in FIG. 12, an embodiment of the present disclosure relates to a method of surface treatment of bone materials by laser and nanostructure thereof. During the osteoblast activation process, the pre-osteoblasts also secrete a variety of proteins, such as COL1A1, OCN and TNFRSF11B. Wherein, the pre-osteoblasts on the surface, which is treated by the laser irradiation group (L), secretes significantly more COL1, OCN and TNFRSF11B. As shown in FIG. 13, the titanium surface treated by the laser irradiation group (L) promotes the osteogenic differentiation markers secretion. The 2.3 × 10⁵ osteoblasts were incubated on the titanium plates treated by G, SLA, and L surface treatments for 72 hours. Cellular medium is collected and used in multiplex assays to determine the concentration of (A) COL1A1, (B) OCN, and (C) TNFRSF11B. Multiple detection data showed that pre-osteoblasts on the surface treated by the laser irradiation group (L) secreted significantly more COL1, OCN and TNFRSF11B. Wherein, osteocalcin (OCN) is a non-collagenous protein secreted by osteoblasts (cells responsible for bone formation) in bone tissue. It plays an important role in bone metabolism and mainly participants in bone mineralization and calcium binding. In addition, osteocalcin (OCN) is also believed to play a role in regulating glucose metabolism and energy balance. Studies have shown that osteocalcin (OCN) is related to insulin secretion, glucose tolerance and adipose tissue function. Osteocalcin (OCN), also known as bone gamma-carboxyglutamic acid-containing protein (BGLAP), is a small non-collagenous hormone found in bone and dentin, which was originally identified as a calcium-binding protein. Osteocalcin is secreted only by osteoblasts and is considered playing a role in the body's metabolic regulation. In its carboxylated form, it binds directly to calcium and thus concentrates in the bones. In its uncarboxylated form, osteocalcin acts as a hormone in the body, sending signals in the pancreas, fat, muscle, testicles, and brain. In the pancreas, osteocalcin acts on beta cells, causing the beta cells in the pancreas to release more insulin. In adipocytes, osteocalcin triggers the release of the hormone adiponectin, which increases sensitivity to insulin. In muscle, osteocalcin acts on myocytes to promote energy availability and utilization and in this way benefits exercise performance. In the testicles, osteocalcin acts on leydig cells to stimulate testosterone biosynthesis, thus affecting male fertility. In the brain, osteocalcin plays important roles in development and function, including spatial learning and memory. The acute stress response (ASR), commonly known as the fight or escape response, stimulates the release of osteocalcin from bones within minutes in mice, rats and humans. In the presence of adrenal insufficiency, injection of high levels of osteocalcin alone can trigger an acute stress response (ASR). In clinic, osteocalcin can be used as a biomarker to assess bone metabolism and to diagnose and monitor bone diseases, such as osteoporosis.

As shown in FIG13, an embodiment of the present disclosure relates to a method of surface treatment of bone materials by laser and nanostructure thereof. The present disclosure may accelerate initial calcification. When pre-osteoblasts are inoculated on the titanium plate, the laser irradiation group (L) shows an increased alizarin red S stain rate on the 18^{th} day, indicating that the surface treated by the laser irradiation group (L) promotes the enhancement of calcium ion secretion. Energy dispersive X-ray spectroscopy is used to detect the precipitation of extracellular minerals near the upper end of the nanostructured microchannel 10. When the surface structure of the present disclosure is embedded in acellular solutions, there is no significant difference in the precipitation of calcium compounds or potassium compounds compared to the surface of the titanium metal 3 or the surface treated by SLA. However, when the osteoblasts are implanted into the titanium plate, the surface structure of the present disclosure shows an increased alizarin red S stain rate on the 18^{th} day, and the calcium ion secretion promoted by the surface structure is enhanced. The surface of the present disclosure has more significant osteocyte mineralization ability than the titanium surface treated by SLA.

As shown in FIG. 13, an embodiment of the present disclosure relates to a method of surface treatment of bone materials by laser and nanostructure thereof. The present disclosure may accelerate initial calcification. The titanium surface treated by the laser irradiation group (L) promotes extracellular mineralization of the pre-osteoblastic cells. (A) Images are collected after culturing the 2.3 × 10⁵ pre-osteoblastic cells on the surfaces of the titanium plates treated by G, SLA, and L for 4 or 18 days. The left column shows a scale of 100 µm, and the right column shows a scale of 50 µm. Reddish-brown calcium compounds indicate the mineralization capacity of pre-osteoblasts. (B) Quantification results are shown by OD540 level. Scales of 100 µm and 50 µm are used for the images in the left and right columns, respectively.

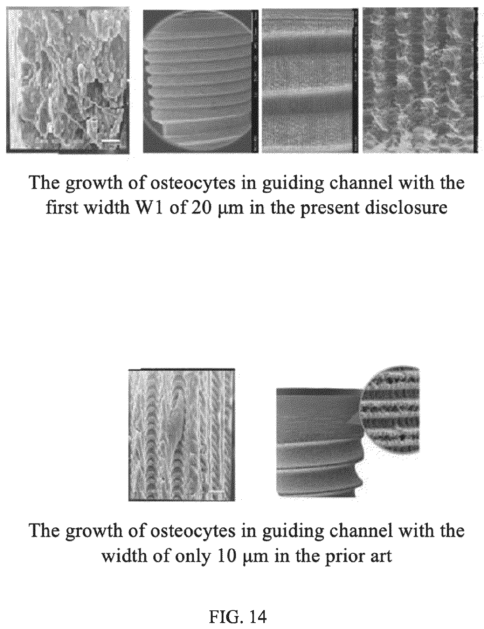

As shown in FIG. 14, an embodiment of the present disclosure relates to a method of surface treatment of bone materials by laser, nanostructure thereof, and the cell morphology and cell adhesion of human fetal osteoblasts (HFOB) implanted on the implant. Lasermodified titanium implants have the potential to increase the chances of successful osseointegration. Because of the microchannels 10 of the present disclosure 10 located in the implant samples with multiple channels and nanostructures, human fetal osteoblasts with filopodia may grow abundantly. The guiding channel 16 of the present disclosure has a sufficient first width W1 of 20 µm to 50 µm, compared with the implant of only 10 µm in the prior art, the surface of the microchannel 10 of the present disclosure has better bone cell growth.

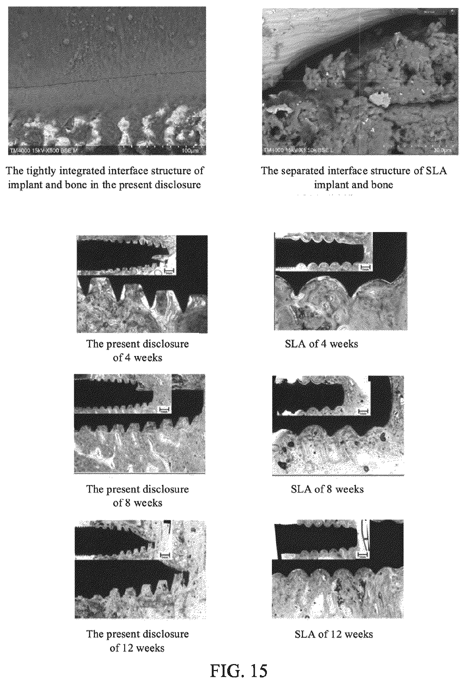

As shown in FIG. 15, an embodiment of the present disclosure relates to a method of surface treatment of bone materials by laser and nanostructure thereof. The present disclosure can stabilize the surface of the bone structure. The initial bone formation starts at the upper edge of the microchannel 10 and the lower edge of the nanostructure. Clinically, the crosssection of present disclosure is taken out from the mouth of a patient in a car accident, and the present disclosure shows a tightly integrated interface. This is completely different from the interface structure of the SLA implant and bone taken out clinically, which the bone and the surface of the SLA implant are separated. The ability of the implant surface integrating with bones is significantly better than that of the SLA implant surface. In addition, animal experiments also show that, within the first two months after implantation, the surface of the implant of the present disclosure has good bone integration ability, while the surface of SLA implant does not show the integration until the third month.

As shown in FIG. 16, an embodiment of the present disclosure relates to a method of surface treatment of bone materials by laser and nanostructure thereof. The present disclosure can resist the formation of dental plaque and has the effect of resisting and inhibiting the growth of bacteria inside the at least one microchannel 10. The nanostructured surface of the microchannel 10 of the present disclosure is compared with a laser surface with another dot matrix pit, a machined surface, a polished surface and a sandblasted surface, a total of five surfaces, regarding the formation and amount of biofilm. The nanostructured surface of the microchannel 10 of the present disclosure may affect the chemical composition of the surface because of the structure of the microchannel 10, causing the surface of the microchannel 10 of the present disclosure form the least biofilm. The microchannel 10 of the present disclosure has a pitch P1 between the guiding channels 16, which prevents the biofilm from crossing the pitch P1 between the guiding channels 16. The present disclosure helps to reduce the occurrence of peri-implantitis. The laser surface of the dot matrix pits in the prior art lacks the pitch P1 between the guiding channels 16, and the biofilm grows and covers different dot matrix pits, so the laser surface of the dot matrix pits in the prior art cannot effectively resist and inhibit the growth of bacteria inside the laser surface of the dot matrix pits.

The surface modification methods in the prior art have the potential risk of introducing foreign matter into the implant surface during the manufacturing process, causing surface contamination and thus reducing safety and effectiveness. By contrast, an embodiment of the present disclosure, which relates to an embodiment of the present disclosure relates to a method of surface treatment of bone materials by laser and nanostructure thereof, is a clean and economical method that does not directly contact the implant during the surface adjustment process, thereby ensuring that the product is not contaminated. In one embodiment of the present disclosure, the structure of the microchannel 10 formed by the titanium metal 3 forms a layer of titanium oxide of titanium dioxide (TiO₂) due to high temperature laser irradiation without any chemical residue. The structure of the nano-microchannel 10 of the present disclosure is at least mainly in a crystalline phase, wherein the crystalline phase has a rutile crystal structure.

Compared the microchannel 10 of the present disclosure with the groups of surfaces treated by the SLA and G, the microchannel 10 of the present disclosure has the nano-bump layer 111 of the nano-wall 11, the wall bottom layer 112 of the nano-wall 11, the micro-holes 12, and the guiding channels 16 located on the surface of the titanium metal 3. The surface structure having nano/micro-holes helps to enhance protein adhesion and cell attachment, provide more climbing points for pre-osteoblast when stretching, and may provide antibiofilm formation ability, promote osteogenic gene expression, such as the expression of parathyroid hormone-related protein gene in osteoblasts, thereby increasing bone integration, osteoblast proliferation and adhesion, proliferation and osteogenic differentiation. The more complex nanoscale structure around the microchannels 10 on the surface of the titanium metal 3 at the laser surface area, the more climbing points are provided for the pre-osteoblasts to stretch, which may promote osteogenic gene expression, such as the expression of parathyroid hormone-related protein genes in osteoblasts. In addition, the microchannels 10 arranged on the deeper titanium surface at the laser surface area of the present disclosure may create more space to promote the full nutritional supplement of pre-osteoblasts and help the pre-osteoblast maturation process, including proliferation, osteogenic differentiation and extracellular mineralization.

The present disclosure provides the microchannels 10 on the titanium surface at the laser surface area, and each process is controlled by specific osteogenic differentiation markers that regulate cellular responses. For example, COL1A1 is upregulated during osteoblast differentiation process, and SPP1 is a marker of extracellular matrix maturation, and DCN regulates collagen matrix assembly and mineralization and modulates the cell cycle and expression of the cell cycle during extracellular matrix mineralization process. In addition, osteoprotegerin translated by the TNFRSF11B gene is a suppressor of bone resorption. Physiological stretching of cells may affect the expression of insulin-like growth factor and mechano-growth factor. Mechanical stretch may detect the mechanosensor polycystin-1 through the mechanism in human osteoblasts. Runx2 expression is promoted through the JAK2/STAT3 pathway. Wherein, JAK-STAT3 is an important signaling pathway involved in regulating cell growth, differentiation and survival. The upregulation of COL1, SPP1, DCN, and TNFRSF11B in human embryonic palatal mesenchymal cells cultured on laser-generated microchannels 10 may be caused by the effect of mechanical stretching forces. The microchannels 10 on the titanium surface of the laser surface area in the present disclosure immediately play a vital role in angiogenesis before the stage of bone integration during implant placement process of mesenchymal fibroblast-like cells and endothelial cells. After the stabilization period, the antimicrobial capacity of the processed implants can indicate the possibility of eliminating peri-implantitis. The microchannels 10 on the titanium surface at the laser surface area of the present disclosure can enhance angiogenesis and antibacterial ability when applied to bone materials. Furthermore, the proliferation of pre-osteoblasts may be enhanced and the expression/secretion of COL1, SPP1, DCN, TNFRSF11B mRNA and protein, as well as extracellular mineralization, may be promoted.

## Claims

1. A method of surface treatment of bone materials by laser, **characterized in that** comprises:
forming molten metallic titanium by continuously ablating a surface of titanium metal of a bone material by irradiating laser at fixed points in a straight axis forward direction along a surface of the titanium metal (3) of the bone material, forming titanium dioxide (TiO₂) during cooling, forming at least one microchannel (10) in the straight axis forward direction by performing molten sputtering on the molten titanium metal (3), wherein the at least one continuous micro-hole (12) is formed at a bottom side of the at least one microchannel (10) by performing molten sputtering at the surface of the molten titanium metal (3) using the laser, at least one nano-wall (11) of the at least one microchannel is formed at a longitudinal axis side of the at least one microchannel by continuously performing molten sputtering at the at least one micro-hole (12) along the straight axis forward direction using the laser and then performing molten sputtering around the at least one micro-hole (12) to sputter the molten titanium metal (3) onto the surface of the titanium metal (3);
forming at least one guiding channel (16) of the at least one microchannel by performing molten sputtering in between the at least one nano-wall (11) in a direction perpendicular to the irradiated straight axis forward direction of the laser, wherein the guiding channel (16) between the adjacent at least one nano-wall (11) has a first width W1 and a thickness of the nano-wall (11) between the paralleled adjacent at least one guide channel has a pitch P1;
forming a first height D1 of the at least one microchannel by irradiating the laser on the surface of the titanium metal (3) of the bone material along a longitudinal axis direction perpendicular to the irradiated straight axis forward direction, wherein the first height D1 comprises a second height D2 of the at least one nano-wall (11), and a third height D3 of a depth of the micro-hole (12);
forming at least one nano-bump layer (111) with clustered nano-bumps on an upper end surface of at least one nano-wall (11) by irradiating the laser on the surface of the titanium metal (3) to perform molten sputtering subject to the surface of the titanium metal (3);
and forming at least one wall bottom layer (112) at a lower end surface of the at least one nano-wall (11) by irradiating the surface of the titanium metal (3) to perform molten sputtering subject to the surface of the titanium metal (3) by the laser;
wherein, a change in roughness is formed by performing molten sputtering by irradiating the laser on the surface of the titanium metal (3), a roughness of the at least one nano-bump layer (111) is greater than a roughness formed by the at least one wall bottom layer (112) of the bottom side of the adjacent guiding channel (16);
wherein, the micro-holes (12) are provided to activate stem cells into osteoblasts, and the osteoblasts form at least one Z-shaped mesh attaching object (15), which quickly climbs the nano-bump layers (111) between the at least one adjacent nano-wall (11) of the at least one guiding channel (16).

2. The method of surface treatment of bone materials by laser according to claim 1, wherein the first width W1 of the guiding channel (16)between adjacent at least one nano-wall (11) is 20 µm to 50 µm, the thickness of the nano-wall (11) between the at least one parallel adjacent guiding channel (16) has the pitch P1 of 10 µm to 30 µm, the first height D1 of at least one microchannel is 20 µm to 60 µm, the second height D2 of the at least one nano-wall (11) is 10 µm to 30 µm, the third height of the depth of the micro-hole (12)is 10 µm to 30 µm, the pitch P1 of the thickness of the at least one nano-wall (11) is 10 µm to 30 µm, a diameter of the at least one micropore is 20 µm to 50 µm, and a spacing of the at least one micro-hole (12) is 30 µm to 50 µm.

3. The method of surface treatment of bone materials by laser according to claim 2, wherein the laser is switched in a giant pulse generator (Q-Switch) mode, and short pulses are output by a solid-state laser to continuously micro-ablating along the straight axis forward direction through a controlled power intensity.

4. The method of surface treatment of bone materials by laser according to claim 1, wherein a protein secretion of pre-osteoblasts formed during an osteoblast activation process of the at least one attaching object (15) is selected from at least one of collagen I type α1 chain (COL1A1), decorin (DCN), tumor necrosis factor receptor superfamily member 11B (TNFRSF11B) and secretory phosphoprotein-1 (SPP1).

5. The method of surface treatment of bone materials by laser according to claim 1, wherein a structure of the at least one microchannel has an ability to resist and inhibit a growth of bacteria inside the at least one microchannel.

6. The method of surface treatment of bone materials by laser according to claim 1, wherein the titanium metal (3) is titanium or titanium alloy, and the microchannel crystal has a rutile crystal structure.

7. The method of surface treatment of bone materials by laser according to claim 1, wherein the bone material is a dental implant (60) or a bone screw.

8. The method of surface treatment of bone materials by laser according to claim 1, wherein the bone material is placed horizontally, and blood is dripped onto a threaded portion (61) of the bone material, resulting in a contact angle α of 0 to 12 degrees.

9. The method of surface treatment of bone materials by laser according to claim 8, wherein the contact angle α of the blood drops to 0 degree in less than 20 seconds.

10. The method of surface treatment of bone materials by laser according to claim 1, wherein a transmission and exchange channel, for nutrients, blood, serum, and bone calcification, is formed in the guiding channel (16) between the at least one adjacent nano-wall (11), the micro-hole (12) of the at least one microchannel forms a microculture dish, the at least one guiding channel (16) and the at least one micro-hole (12) form a simulated biological environment of the at least one microchannel to promote the formation of new blood vessels.

11. A nanostructure of surface treatment of bone materials by laser, wherein a surface of a bone material is titanium metal (3), molten metallic titanium is formed by continuously ablating a surface of the titanium metal (3) of the bone material by irradiating laser at fixed points in a straight axis forward direction along a surface of the titanium metal (3) of the bone material, titanium dioxide (TiO₂) is formed during cooling, at least one microchannel in the straight axis forward direction is formed by performing molten sputtering on the molten titanium metal (3), the at least one microchannel, **characterized in that** comprises:
at least one micro-hole (12) arranged on a bottom side of the at least one microchannel along the straight axis forward direction of the laser, wherein the laser continuously melts and sputters the surface of the titanium metal (3) to form the at least one continuous micro-hole (12) of the at least one microchannel;
at least one nano-wall (11) arranged on a longitudinal axis side of the at least one microchannel formed along the straight axis forward direction of the laser, wherein after the laser continuously melts and sputters the at least one micro-hole (12), the laser melts and sputters around the at least one micro-hole (12) onto the surface of the titanium metal (3) to form the at least one nano-wall (11) of the at least one microchannel, on the upper end surface of the at least one nano-wall (11), at least one nano-bump layer (111) with clustered nano-bumps is formed by melting and sputtering the surface of the titanium metal (3), and on the lower end surface of the at least one nano-wall (11), at least one wall bottom layer (112) is formed by melting and sputtering on the surface of the titanium metal (3);
at least one guiding channel (16) arranged between the adjacent at least one nano-wall (11), wherein, by irradiating the laser along a vertical direction of the irradiated straight axis forward, the laser melts and sputters between the adjacent at least one nano-wall (11) to form the at least one guiding channel (16) of the at least one microchannel;
wherein, the guiding channel (16) between the adjacent at least one nano-wall (11) has a first width W1 and a thickness of the nano-wall (11) between the parallel adjacent at least one guiding channel (16) has a pitch P1;
wherein a first height D1 of the at least one microchannel comprises a second height D2 of the at least one nano-wall (11) and a third height of a depth of the micro-hole (12);
wherein, a change in roughness is formed by performing molten sputtering through irradiating the laser on the surface of the titanium metal (3), a roughness of the at least one nano-bump layer (111) is greater than a roughness formed by the at least one wall bottom layer (112) of the bottom side of the adjacent guiding channel (16);
wherein the micro-holes (12) are provided to activate stem cells into osteoblasts, and the osteoblasts form at least one Z-shaped mesh attaching object (15), which quickly climbs the nano-bump layers (111) of the at least one nano-wall (11) between the adjacent at least one guiding channel (16).

12. The nanostructure of surface treatment of bone materials by laser according to claim 11, wherein the first width W1 of the guiding channel (16) between adjacent at least one nano-wall (11) is 20 µm to 50 µm, the thickness of the nano-wall (11) between the at least one parallel adjacent guiding channel (16) has the pitch P1 of 10 µm to 30 µm, the first height D1 of at least one microchannel is 20 µm to 60 µm, the second height D2 of the at least one nano-wall (11) is 10 µm to 30 µm, the third height of the depth of the micro-hole (12) is 10 µm to 30 µm, the pitch P1 of the thickness of the at least one nano-wall (11) is 10 µm to 30 µm, a diameter of the at least one micropore is 20 µm to 50 µm, and a spacing of the at least one micro-hole (12) is 30 µm to 50 µm.

13. The nanostructure of surface treatment of bone materials by laser according to claim 12, wherein the laser is switched in a giant pulse generator (Q-Switch) mode, and short pulses are output by a solid-state laser to continuously micro-ablating along the straight axis forward direction through a controlled power intensity.

14. The nanostructure of surface treatment of bone materials by laser according to claim 11, wherein a protein secretion of pre-osteoblasts formed during an osteoblast activation process of the at least one attaching object (15) is selected from at least one of collagen I type α1 chain (COL1A1), decorin (DCN), tumor necrosis factor receptor superfamily member 11B (TNFRSF11B) and secretory phosphoprotein-1 (SPP1).

15. The nanostructure of surface treatment of bone materials by laser according to claim 11, wherein a structure of the at least one microchannel has an ability to resist and inhibit a growth of bacteria inside the at least one microchannel.

16. The nanostructure of surface treatment of bone materials by laser according to claim 11, wherein the titanium metal (3) is titanium or titanium alloy, and the microchannel crystal has a rutile crystal structure.

17. The nanostructure of surface treatment of bone materials by laser according to claim 11, wherein the bone material is a dental implant (60) or a bone screw.

18. The nanostructure of surface treatment of bone materials by laser according to claim 11, wherein the bone material is placed horizontally, and the blood is dripped onto the threaded portion (61)of the bone material, resulting in a contact angle α of 0 to 12 degrees.

19. The nanostructure of surface treatment of bone materials by laser according to claim 18, wherein the contact angle α of the blood drops to 0 degree in less than 20 seconds.

20. The nanostructure of surface treatment of bone materials by laser according to claim 11, wherein a transmission and exchange channel, for nutrients, blood, serum, and bone calcification, is formed in the guiding channel (16) between the at least one adjacent nano-wall (11), the micro-hole (12) of the at least one microchannel forms a microculture dish, the at least one guiding channel (16) and the at least one micro-hole (12) form a simulated biological environment of the at least one microchannel to promote the formation of new blood vessels.
